Europäisches Patentamt

European Patent Office

Office européen des brevets

(11) Publication number: 0 289 199 A1

# EUROPEAN PATENT APPLICATION

(21) Application number: 88303532.1

(22) Date of filing: 20.04.88

(51) Int. Cl.⁴: A61B 5/00

(30) Priority: 28.04.87 GB 8709986

(43) Date of publication of application:
02.11.88 Bulletin 88/44

(84) Designated Contracting States:
DE ES FR IT NL SE

(71) Applicant: The BOC Group plc
Chertsey Road
Windlesham Surrey GU20 6HJ(GB)

(72) Inventor: Dickens, Robert Andrew
4 Salop Close Shrivenham
Nr. Swindon Wiltshire SN6 8DN(GB)
Inventor: Scott, Ian Lawrence
11 Stirling Close Wroughton
Swindon Wiltshire SN4 9HG(GB)
Inventor: Kratochvil, Jiri
1281 East Ridgemeadow Lane
No 8E Midvale Utah 84087(US)

(74) Representative: Gough, Peter
c/o THE BOC GROUP PLC Patent Department
Chertsey Road
Windlesham Surrey GU20 6HJ(GB)

(54) Improvements in sensors.

(57) A sensor for measuring gas partial pressures in fluids such as blood comprises a probe 2 which is occluded at its distal end by a dome 3 of insulating material. Embedded in the dome 3 are electrodes and covering the outer surface of the dome is a membrane. The membrane comprises a first inner layer of hydrophilic material and a second outer layer of hydrophobic material. The outer layer is selectively permeable so that water and small ionic species from the blood pass through the outer layer into the inner layer to form a gel electrolyte.

EP 0 289 199 A1

FIG. 2.

## IMPROVEMENTS IN SENSORS

The present invention relates to intravascular sensors and in particular, to electrochemical sensors for measuring the partial pressure of oxygen in blood.

Electrochemical sensors are known which comprise a pair of electrodes and an electrolytic medium in contact with both electrodes which form together an electrochemical cell. The cell is separated from the solution, for example, blood to be investigated by a membrane which is permeable to oxygen. Thus, in use oxygen diffuses into the cell through the membrane so as to affect the characteristics of the cell to an extent dependent on the concentration of oxygen in the solution. Hydrophobic membranes have been used to protect the cell and, in particular, the electrodes from contaminants such as proteins when blood is being investigated. The problem with such hydrophobic membranes is that the sensor must be assembled with a fluid electolyte in place, prior to applying the membrane. A wet device is less easy to manufacture than a dry device and has a shorter shelf life.

It is an aim of the present invention to provide an intravascular sensor having a probe with a tip a major portion of which is covered by a double layer membrane to produce a smooth haemo-compatible coating.

It is a further aim of the present invention to provide a dry sensor which activates rapidly once placed in a suitable fluid (blood or a fluid with a similar ionic composition).

It is yet a further aim of the present invention to provide a miniaturised sensor for measuring the partial pressure of oxygen in a solution, for example, blood which has improved stability, repeatability and a faster reaction time in-vivo than prior art sensors.

According to the present invention, a sensor for measuring gas partial pressures in fluids comprises a hollow tubular probe, the distal end of which probe is occluded by a dome of insulating material, said dome defining the tip of the probe, at least first and second electrodes disposed in the dome at spaced apart locations, and at least a major portion of the dome being covered by a membrane comprising a first inner layer of a hydrophilic material and a second outer layer of hydrophobic material, the outer layer being selectively permeable so that when the sensor is exposed to fluid the outer layer allows the passage of water and small ionic species from the fluid into the first inner layer to form a gel electrolyte

An embodiment of the invention will now be described, by way of example, reference being made to the Figures of the accompanying diagrammatic drawings in which :-

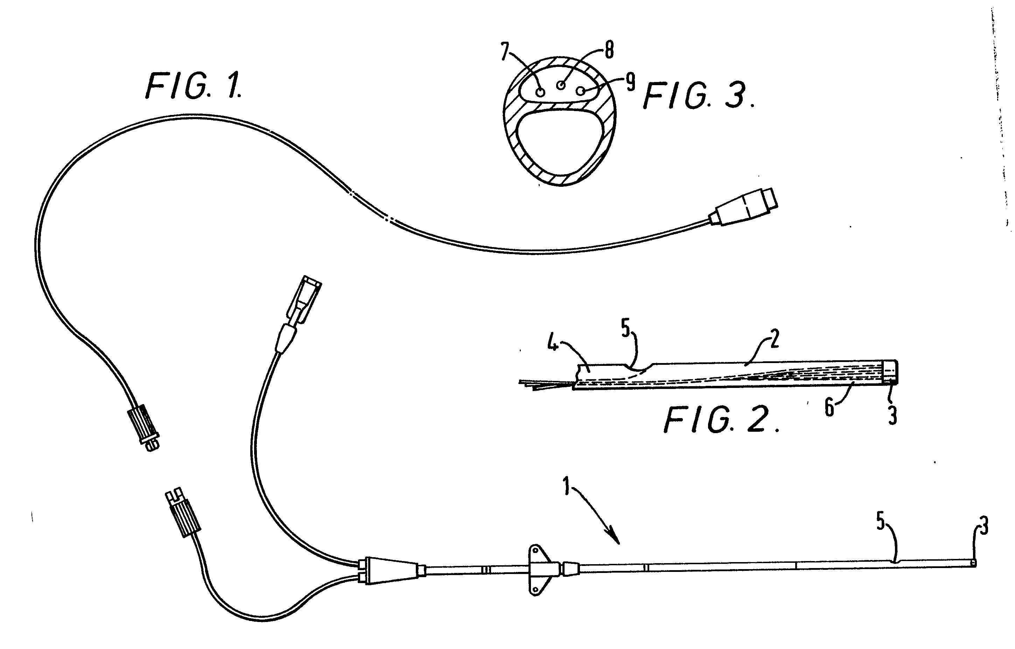

Figure 1 is a plan view of a sensor for measuring the partial pressure of oxygen in blood; .

Figure 2 is an enlargement of a probe forming part of the sensor of Figure 1; and

Figure 3 is a transverse cross-section through the probe of Figure 2.

As shown, a sensor 1 for measuring the partial pressure of oxygen in the blood of a patient includes a hollow tubular probe 2 suitable for insertion into a blood vessel of a patient. At its distal end the probe 2 is occluded by a dome 3 of insulating material which is shaped and polished to define the tip of the probe 2.

The probe 2 may be made, for example, of a medical grade plastics such as polyurethane and the insulating material may be made from an epoxy resin.

The probe 2 has two lumens, a first lumen 4 which terminates at an outlet 5 spaced from the dome 3 and a second lumen 6. The first lumen 4 is an infusion lumen for the supply to the patient of drugs and the like whereas the lumen 6 accommodates three conductors 7, 8 and 9 each connected to an associated electrode, the electrodes being disposed in the dome 3 at spaced apart locations. The three electrodes include a cathode made of silver, a reference electrode made of silver chloride and an anode made of platinum.

The outer surface of the dome 3 is covered with a membrane comprising a first inner layer of a very hydrophilic material for example a hydrogel. One such hydrogel is the reaction product of a moisture curable isocyanate prepolymer with high molecular weight, nonionic polyvinylpyrrolidone, (PVP). The solution also contains a fluorosurfactant to facilitate a uniform wetting of the dome and the catheter tubing during the coating. Proper crosslink density of the interpolymer, which depends on the ratio of the prepolymer to PVP, is very important because it substantially influences the sensors performance.

Generally, suitable polymeric materials for the outer layer of the membrane coating are those which exhibit high tensile strengths when cured and of high flexibility, prefereably Hardness Shore A 83. The films formed from these solutions will also have some degree of permeability to water, as well as being permable to small inorganic ions. When introduced into a suitable fluid water passes across the outer layer and hydrates the inner layer, taking small ionic species with it, thus forming a gel electrolyte. The outer layer controls the rate of flux from the fluid into the inner layer and therefore the activation time of the sensor. Once the sensor is activated the outer layer in conjunction with the inner gel acts as a diffusion barrier to oxygen, and therefore controls the current output, response time and stability of the sensor. The outer layer also has the important task of acting as an interface with the blood, in these circumstances the chemistry of the membrane surface and its topography are important.

Suitable outer layers are silicone thermoplastic copolymer PS 254 MB as supplied by Prarch Systems Inc. 6.6% - 8% solution in solvent and Tecoflex SG-83A castable grade (Thermedics Inc) as a 4% solution.

For the inner hydrogel, the effect of different crosslink density as well as the effect of different solids content was investigated by polarography (see tables 1, 2 and 3). In table 3 the results are shown for an outer membrane layer of a silicone thermoplastic copolymer of 6.6%-8% solids content (Prarch Systems Inc.)

## Table 1

| PVP Content (%) | Cross-link density | Appearance |
|---|---|---|
| 1.5 | #1 | clear |
| 1.5 | #2 | clear |
| 1.5 | #3 | clear |
| 3.0 | #1 | clear |
| 3.0 | #2 | clear |
| 3.0 | #3 | opaque/precipate |
| 3.0 | #4 | opaque/precipate |
| 6.0 | #1 | clear/viscous |
| 6.0 | #2 | clear/gel |
| 6.0 | #3 | clear/gel |

The crosslink density increases from #1 to #4.

## Table 2

| Crosslink Density | PVP/isocyanate prepolymer by weight |
|---|---|
| #1 | 80/20 |
| #2 | 67/33 |
| #3 | 58/42 |
| #4 | 50/50 |

### Table 3

| Inner coat PVP/crosslink density | current (nA) at −600 mV with reference to Ag/Ag Cl |
|---|---|
| 1.5%,#1 | 8.2 & 9.9 |
| 1.5%,#2 | 4.6 & 8.6 |
| 3.0%,#1 | 8.0 & 6.1 |
| 3.0%,#2 | 2.6 & 2.9 |
| 6.0%,#1 | 2.6 & 2.2 |

As can be seen, the increased amount of the isocyanate prepolymer and PVP lowers the current. Low solids (1.5%) solutions are too thin and the high solids (6%) solutions are subject to ageing and are difficult to handle. For these reasons the preferred composition of the inner coating is 3%,#1, PVP, K-90.

Although, reference has been made in the above description to an electrochemical sensor for measuring the partial pressure of oxygen in the blood of a patient, the double layer membrane can be applied to other types of intravascular sensors, for example, optical or silicon chip sensors.

## Claims

1. A sensor for measuring gas partial pressures in fluids comprising a hollow tubular probe 2, the distal end of which probe is occluded by a dome 3 of insulating material, said dome 3 defining the tip of the probe, characterised in that at least first and second electrodes are disposed in the dome 3 at spaced apart locations, and at least a major portion of the dome 3 is covered by a membrane comprising a first inner layer of a hydrophilic material and a second outer layer of hydrophobic material, the outer layer being selectively permeable so that when the sensor is exposed to fluid the outer layer allows the passage of water and small ionic species from the fluid into the first inner layer to form a gel electrolyte

2. A sensor as claimed in claim 1, characterised in that the second layer is a hydrophobic linear polyether polyurethane.

3. A sensor as claimed in claim 1 or 2, characterised in that the first layer is a hydrogel.

4. A sensor as claimed in claim 3, characterised in that the hydrogel is crosslinked, high molecular weight nonionic, polymer, polyvinyl pyrrolidone dissolved in methylethylketone, ethyl lactate and trichloroethylene.

5. A sensor as claimed in claim 4, characterised in that the solution includes a moisture curable isocyanate prepolymer and a fluorosurfactant.

FIG. 1.

FIG. 2.

FIG. 3.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO-A-8 102 218 (J.A.R. KATER) * abstract; page 16, line 21 - page 17, line 11; page 13, line 30 - page 14, line 10, figures 1-3 * | 1,3 | A 61 B 5/00 |
| A | US-A-3 856 649 (M.A. GENSHAW et al.) * abstract; column 1, line 60 - column 3, line 29 * | 1-5 | |
| A | GB-A-1 253 492 (BECKMAN INSTRUMENTS, INC.) * page 3, line 17 - page 4, line 36; figure 1 * | 1-5 | |
| A | EP-A-0 050 035 (SMITH AND NEPHEW ASSOCIATED COMPANIES LTD.) * page 11, lines 16-20 * | 2 | |

TECHNICAL FIELDS
SEARCHED (Int. Cl.4)

A 61 B 5/00
G 01 N 27/00

The present search report has been drawn up for all claims

| Place of search | Date of completion of the search | Examiner |
|---|---|---|
| BERLIN | 25-07-1988 | WEIHS J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)